(19) Europäisches Patentamt
European Patent Office
Office européen des brevets

(11) Veröffentlichungsnummer: **0 086 424**
**B1**

(12) # EUROPÄISCHE PATENTSCHRIFT

(45) Veröffentlichungstag der Patentschrift: **12.09.84**

(51) Int. Cl.³: **C 07 C 118/00**

(21) Anmeldenummer: **83101129.1**

(22) Anmeldetag: **07.02.83**

(54) **Verfahren zur Herstellung von 1-Alkenylisocyanaten.**

(30) Priorität: **16.02.82 DE 3205433**

(43) Veröffentlichungstag der Anmeldung: **24.08.83 Patentblatt 83/34**

(45) Bekanntmachung des Hinweises auf die Patenterteilung: **12.09.84 Patentblatt 84/37**

(84) Benannte Vertragsstaaten: **BE CH DE FR GB IT LI NL**

(56) Entgegenhaltungen:
**EP-A-0 025 552**
**EP-A-0 025 906**
**FR-A-1 172 767**

(73) Patentinhaber: **BASF Aktiengesellschaft, Carl-Bosch-Strasse 38, D-6700 Ludwigshafen (DE)**

(72) Erfinder: **Schwendemann, Volker, Dr., Hauptstrasse 64 A, D-6901 Wiesenbach (DE)**
Erfinder: **Koenig, Karl-Heinz, Dr., Pierstrasse 8 A, D-6710 Frankenthal (DE)**

**EP 0 086 424 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 1-Alkenylisocyanaten durch Umsetzung von 1-Halogenalkylcarbamidsäurehalogeniden oder 1-Halogenalkylisocyanaten mit Carbonsäureamiden.

Die wirtschaftliche Synthese von 1-Alkenylisocyanaten ist wegen der grossen Reaktivität dieser Verbindungen schwierig, da 1-Alkenylisocyanate nicht nur thermisch labil, sondern auch säure-, basen- und hydrolyseempfindlich sind. Die bekanntesten Herstellungsmethoden sind der Curtius-Abbau von substituierten Acrylsäureaziden [J. Org. Chem., Band 26 (1961), Seiten 770 bis 779], die Vakuumpyrolyse von Trisvinylisocyanuraten (DE-AS Nr. 1932811) und die thermische Spaltung von N-tert.-Alkyl-N-(1-alkenyl)-carbamidsäurechloriden (DE-AS Nr. 1922412).

Aus DE-OS Nr. 2937028 ist bekannt, Gemische von 1-monohalogenierten Isocyanaten und 1-Alkenylisocyanaten durch Umsetzung von 1-Halogenalkylcarbamidsäurehalogeniden mit höhersiedenden Isocyanaten darzustellen. Dabei fallen jedoch die 1-Alkenylisocyanate nachteilig nicht in reiner Form an.

Aus DE-OS Nr. 2937006 ist bekannt, dass man aus 1-Halogenalkylcarbamidsäurehalogeniden oder 1-Halogenalkylisocyanaten durch Umsetzung mit α-Pinen ein Gemisch aus 1-Halogenalkylisocyanaten und 1-Alkenylisocyanaten erhält. Nachteilig ist, dass das α-Pinen sich in Bornylchlorid umlagert und somit nicht regeneriert werden kann.

Es wurde nun gefunden, dass man 1-Alkenylisocyanate der Formel

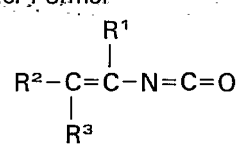 (I)

worin $R^1$, $R^2$ und $R^3$ gleich oder verschieden sein können und jeweils ein Wasserstoffatom oder einen Methylrest bedeuten, vorteilhaft erhält, wenn man 1-Halogenalkylcarbamidsäurehalogenide der Formel

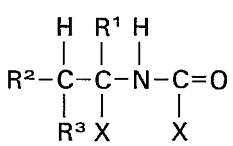 (II)

und/oder 1-Halogenalkylisocyanate der Formel

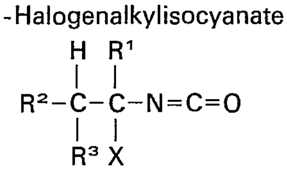 (III)

worin $R^1$, $R^2$ und $R^3$ die vorgenannte Bedeutung besitzen, und X für ein Halogenatom steht, mit Carbonsäureamiden der Formel

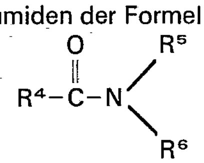 (IV)

worin $R^4$ für ein Wasserstoffatom, einen aliphatischen oder aromatischen Rest steht, $R^5$ und $R^6$ gleich oder verschieden sein können und jeweils ein Wasserstoffatom oder einen aliphatischen Rest bedeuten oder zusammen mit dem benachbarten Stickstoffatom für Glieder eines heterocyclischen Restes stehen, umsetzt.

Die Umsetzung kann für den Fall der Verwendung von 1-Chlorethylcarbamidsäurechlorid und N,N-Dimethylacetamid durch die folgenden Formeln wiedergegeben werden

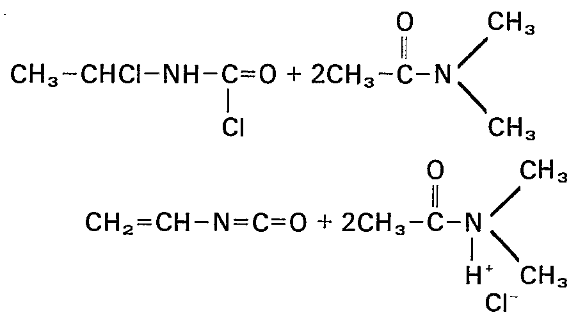

Im Hinblick auf den Stand der Technik liefert das Verfahren nach der Erfindung überraschend auf einfacherem und wirstschaftlicherem Wege 1-Alkenylisocyanate in guter Ausbeute und Reinheit. Diese vorteilhaften Eigenschaften des erfindungsgemässen Verfahrens waren nicht vorauszusehen, denn es ist aus „The Chemistry of Cyanates and their Thioderivatives" [Saul Patai, John Wiley & Sons, New York (1977), Seite 674] bekannt, dass organische Isocyanate bei der Behandlung mit Base leicht unter Bildung von Isocyanuraten trimerisieren. Aus Synthesis Band *1980*, Seite 85, ist bekannt, dass α-Halogenisocyanate eine weitere verstärkte Reaktivität der Isocyanatfunktion und des α-Halogenatoms aufweisen. Es ist ebenfalls überraschend, dass man mit Carbonsäureamiden IV auch Halogenwasserstoff, der nicht von der Carbamidsäurehalogenidgruppe sondern von den Atomen des Kohlenstoffgerüstes stammt, unter Ausbildung einer olefinischen Doppelbindung eliminieren kann. Das erfindungsgemässe Verfahren ist um so überraschender, da 1-Alkenylisocyanate bekanntlich ausserordentlich empfindlich gegen Halogenwasserstoff sind und schon bei Raumtemperatur mit diesem harzartige Polymersubstanzen bilden [Recueil, Band 94 (1975), Seite 102] und bei sehr niederen Temperaturen diesen spontan addieren (DE-OS Nr. 2732284). In Anbetracht des Carbamidsäurechlorid-Isocyanat-Gleichgewichts (Houben-Weyl, Methoden der Organischen Chemie, Band 8, Seite 121) war zu erwarten gewesen, dass entstandenes 1-Alkenylisocyanat mit Halogenwasserstoff aus noch nicht umgesetztem 1-Halogenalkylcarbamidsäurehalogenid zu polymeren Verbindungen abreagieren würde.

Bevorzugte Ausgangsstoffe II, III und IV und demzufolge bevorzugte Endstoffe I sind solche, in deren Formeln die Reste X gleich oder verschieden im Ausgangsstoff II sein können und vorteilhaft ein Bromatom oder insbesondere ein Chloratom bedeuten, $R^4$ für ein Wasserstoffatom, einen Alkylrest mit 1 bis 12, vorzugsweise 1 bis 6 Kohlenstoffatomen oder einen Phenylrest steht, $R^5$ und $R^6$ gleich oder verschieden sein können und jeweils ein Wasserstoffatom oder einen Alkylrest mit 1 bis 6 Kohlenstoffatomen bedeuten oder zusammen mit dem benachbarten Stickstoffatom für Glieder eines 5- bis 8gliedrigen heterocyclischen Ringes, der neben dem Stickstoffatom noch ein weiteres Stickstoffatom oder ein Sauerstoffatom enthalten kann, stehen. Die vorgenannten Reste und Ringe können noch durch unter den Reaktionsbedingungen inerte Gruppen oder Atome, z.B. Alkyl-, Alkoxygruppen mit jeweils 1 bis 4 Kohlenstoffatomen, die Phenylgruppe substituierende Chloratome, substituiert sein. Die Ausgangsstoffe II werden auf einfache Weise, z.B. nach dem in der DE-OS Nr. 2741980 beschriebenen Verfahren, hergestellt.

Geeignete Ausgangsstoffe II sind beispielsweise: 1-Chlorethylcarbamidsäurechlorid, 1-Bromethylcarbamidsäurebromid, 1-Chlorpropylcarbamidsäurechlorid, 1-Brompropylcarbamidsäurebromid, 1-Chlor-1-methylethylcarbamidsäurechlorid, 1-Brom-1-methylethylcarbamidsäurebromid, 2-Chlorbutyl-(2)-carbamidsäurechlorid, 2-Brombutyl-(2)-carbamidsäurebromid, 1-Chlor-2-methylpropylcarbamidsäurechlorid, 1-Brom-2-methylpropylcarbamidsäurebromid, 2-Chlor-3-methylbutyl-(2)-carbamidsäurechlorid, 2-Brom-3-methylbutyl-(2)-carbamidsäurebromid.

Geeignete Ausgangsstoffe III sind beispielsweise: 1-Chlorethylisocyanat, 1-Bromethylisocyanat, 1-Chlorpropylisocyanat, 1-Brompropylisocyanat, 1-Chlor-1-methylethylisocyanat, 1-Brom-1-methylethylisocyanat, 2-Chlorbutyl-(2)-isocyanat, 2-Brombutyl-(2)-isocyanat, 1-Chlor-2-methylpropylisocyanat, 1-Brom-2-methylpropylisocyanat, 2-Chlor-3-methylbutyl-(2)-isocyanat, 2-Brom-3-methylbutyl-(2)-isocyanat.

Die Umsetzung wird in der Regel bei einer Temperatur von −10 bis 150°C, vorzugsweise 30 bis 120°C, drucklos oder unter Druck, kontinuierlich oder diskontinuierlich durchgeführt. Vorteilhaft beginnt man die Umsetzung bei einer Temperatur von −10 bis 30°C, erhöht langsam die Temperatur und beendet die Reaktion bei den vorgenannten, bevorzugten Reaktionstemperaturen. Die Reaktionszeit beträgt im allgemeinen 0,5 bis 6 Stunden.

Die Menge an Ausgangsstoff IV wird zweckmässig entsprechend der Menge des abzuspaltenden Halogenwasserstoffes bzw. der gewünschten Menge an Endstoff I gewählt. So verwendet man vorteilhaft 1 bis 20, bevorzugt 2 bis 6 Mol Carbonsäureamid IV pro Mol Ausgangsstoff II und 1 bis 10, bevorzugt 1 bis 5 Mol Carbonsäureamid IV pro Mol Ausgangsstoff III.

Bevorzugt setzt man in Abwesenheit von Lösungsmitteln um, jedoch können auch unter den Reaktionsbedingungen inerte organische Lösungsmittel verwendet werden. Als Lösungsmittel können z.B. verwendet werden: Aliphatische oder cycloaliphatische Kohlenwasserstoffe wie Pentan, Nonan, Heptan, Octan, Cyclohexan; halogenierte aliphatische Kohlenwasserstoffe wie Methylenchlorid, Chloroform, Tetrachlorkohlenstoff, n-Propylchlorid, n-Butylchlorid und Isomere, Amylchlorid, Cyclohexylchlorid, Ethylidenchlorid, Dichlorethylen, Ethylenchlorid, Dichlorpropan, Dichlorbutan, Tetrachlormethan, Isopropylbromid, n-Propylbromid, Butylbromid, Ethyljodid, Propyljodid sowie fluorierte oder teilweise fluorierte Verbindungen wie Hexylfluorid, Trichlortrigluorethan; aromatische Kohlenwasserstoffe wie Benzol, Toluol, Xylol, Ethylbenzol, Chlorbenzol, Dichlorbenzol, Fluorbenzol, Difluorbenzol, Nitrobenzol; cyclische Ether wie Dioxan, Tetrahydrofuran; Naphthalinderivate wie Chlornaphthalin; Ketone wie Aceton, Methylethylketon, Diethylketon Acetophenon; Ester wie Ameisensäureethylester, Essigsäuremethylester, Propionsäureethylester, Phthalsäuremethylester, Schwefelkohlenstoff, Methyl-tert.-butylether, Ethylpropylether, Acetonitril; und entsprechende Gemische. Vorzugsweise werden Chlorbenzol, Tetrachlormethan oder Ethylenchlorid verwendet. Zweckmässig verwendet man das Lösungsmittel in einer Menge von 50 bis 10000 Gew.-%, vorzugsweise von 50 bis 1000 Gew.-%, bezogen auf Ausgangsstoff II oder III.

Die Reaktion kann wie folgt durchgeführt werden: Ein Gemisch von Ausgangsstoff II oder III und IV wird bei der Reaktionstemperatur während vorgenannter Reaktionszeit umgesetzt. Aus dem Reaktionsgemisch wird dann der Endstoff I, zweckmässig direkt nach der Reaktion durch Erhöhung der Temperatur und Destillation, abgetrennt. Anstelle der reinen 1-Halogenalkylisocyanate III oder 1-Halogenalkylcarbamidsäurehalogenide II lassen sich auch die Reaktionsgemische aus der Herstellung dieser Ausgangsstoffe, z.B. die rohen Halogenierungsgemische, verwenden, die bei der Halogenierung von Carbamidsäurehalogeniden anfallen.

Die erfindungsgemäss herstellbaren 1-Alkenylisocyanate I sind wertvolle Ausgangsstoffe für die Herstellung von Schädlingsbekämpfungsmitteln, Farbstoffen, Pharmazeutika, Textilhydrophobierungsmitteln, Waschmitteln, Kunststoffen, Bleichmitteln und Klebstoffen. Zudem sind 1-Alkenylisocyanate wichtige Monomere, die zu Ketten- und Leiterpolymeren, wie z.B. strahlungshärtenden Lackharzen, umgesetzt werden können [C.A., *51*, (1957), 18694 b-e; J. Polymer Sci., *35*, (1959), Seiten 215 bis 218, J. of Coatings Techn., *49*, (1977), Heft 632, Seiten 82 bis 86]. Sie können zu Urethanen, z.B. für die Verwendung als Schaumstoffe oder hochmolekulare Überzüge mit hoher Flexibilität, oder Harnstoffen umgesetzt werden. Bezüglich der Verwendung wird auf vorgenannte Veröffentlichungen und Ullmanns Enzyklopädie der technischen Chemie, Band 9, Seiten 11, 12, 404 sowie Band 17, Seite 204 (3. Auflage), hingewiesen.

*Beispiel 1*

Zu 142 g α-Chlorethylcarbamidsäurechlorid wurden 348 g N,N-Dimethylacetamid getropft. Man erwärmte das Gemisch auf 40°C und rührte 20 Min, wobei sich ein festes Gemisch bildete.

Dann wurde das Gemisch langsam (60 Min) auf 80° C erwärmt und das entstandene Vinylisocyanat bei 100 mbar abdestilliert. Man erhielt 54,5 g Vinylisocyanat vom Fp 38 bis 40° C in einer Reinheit von über 98%, entsprechend einer Ausbeute von 79% der Theorie.

*Beispiel 2*

Zu 142 g α-Chlorethylcarbamidsäurechlorid in 172 g Chlorbenzol wurden 348 g N,N-Dimethylacetamid gegeben. Man erwärmte das Gemisch auf 40° C und destillierte anschliessend bei 70° C und 100 mbar das Vinylisocyanat ab. Man erhielt 57,3 g Vinylisocyanat vom Fp 38 bis 40° C in einer Reinheit von über 98%, entsprechend einer Ausbeute von 83% der Theorie.

*Beispiel 3*

Zu 231 g α-Bromethylcarbamidsäurebromid wurden 348 g N,N-Dimethylacetamid getropft. Man erwärmte das Gemisch auf 40° C und rührte 20 Min, wobei sich ein festes Gemisch bildete. Dann wurde das Gemisch langsam (60 Min) auf 110° C erwärmt und das entstandene Vinylisocyanat bei 100 mbar abdestilliert. Man erhielt 53,1 g Vinylisocyanat vom Fp 38 bis 40° C in einer Reinheit von über 99%, entsprechend einer Ausbeute von 77% der Theorie.

## Patentanspruch

Verfahren zur Herstellung von 1-Alkenylisocyanaten der Formel

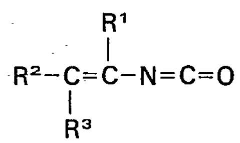 (I)

worin $R^1$, $R^2$ und $R^3$ gleich oder verschieden sein können und jeweils ein Wasserstoffatom oder einen Methylrest bedeuten, dadurch gekennzeichnet, dass man 1-Halogenalkylcarbamidsäurehalogenide der Formel

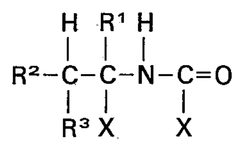 (II)

und/oder 1-Halogenalkylisocyanate der Formel

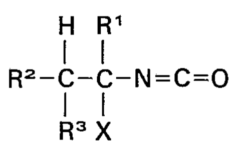 (III)

worin $R^1$, $R^2$ und $R^3$ die vorgenannte Bedeutung besitzen, und X für ein Halogenatom steht, mit Carbonsäureamiden der Formel

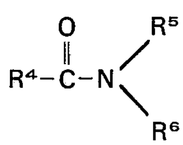 (IV)

worin $R^4$ für ein Wasserstoffatom, einen aliphatischen oder einen aromatischen Rest steht, $R^5$ und $R^6$ gleich oder verschieden sein können und jeweils ein Wasserstoffatom oder einen aliphatischen Rest bedeuten oder zusammen mit dem benachbarten Stickstoffatom, für Glieder eines heterocyclischen Restes stehen, umsetzt.

## Claim

A process for the preparation of an alk-1-enyl isocyanate of the formula

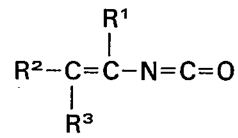 (I)

where $R^1$, $R^2$ and $R^3$ may be identical or different and are each hydrogen or methyl, wherein a 1-haloalkylcarbamyl halide of the formula

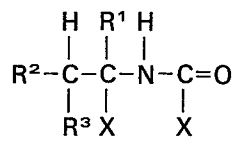 (II)

and/or a 1-haloalkyl isocyanate of the formula

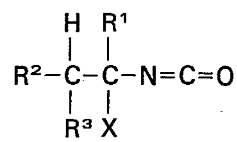 (III)

where $R^1$, $R^2$ and $R^3$ have the above meanings and X is halogen, are reacted with a carboxamide of the formula

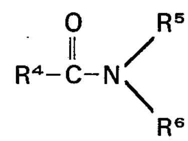 (IV)

where $R^4$ is hydrogen or an aliphatic or aromatic radical, and $R^5$ and $R^6$ may be identical or different and are each hydrogen or an aliphatic radical, or, together with the adjacent nitrogen atom, are members of a heterocyclic radical.

## Revendication

Procédé de préparation d'isocyanates de 1-alcényle de la formule

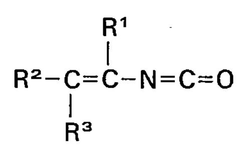 (I)

dans laquelle $R^1$, $R^2$ et $R^3$, qui peuvent être identiques ou différents, désignent chacun un atome d'hydrogène ou un radical méthyle, caractérisé en ce que l'on fait réagir des halogénures d'acide 1-haloalkylcarbamique de la formule

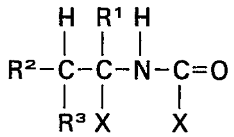

(II)

et/ou des isocyanates de 1-haloalkyle de la formule

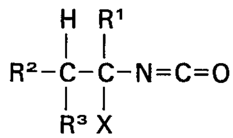

(III)

dans lesquelles $R^1$, $R^2$ et $R^3$ possèdent la signification définie et X désigne un atome d'halogène, avec des amides d'acides carboxyliques de la formule

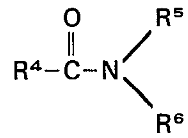

(IV)

dans laquelle $R^4$ désigne un atome d'hydrogène ou un groupe aliphatique ou aromatique et $R^5$ et $R^6$, qui peuvent être identiques ou différents, désignent chacun un atome d'hydrogène ou un groupe aliphatique ou forment avec l'atome d'azote adjacent des maillons d'un groupe hétérocyclique.